Europäisches Patentamt

European Patent Office

Office européen des brevets

(11) Publication number: **0 223 745**
**A1**

## EUROPEAN PATENT APPLICATION

(21) Application number: **86830316.5**

(22) Date of filing: **31.10.86**

(51) Int. Cl.⁴: **A 61 F 13/20**
**A 61 B 10/00**

(30) Priority: **15.11.85 IT 525385**

(43) Date of publication of application:
**27.05.87 Bulletin 87/22**

(84) Designated Contracting States:
**AT BE CH DE FR GB LI LU NL**

�<br>71 Applicant: **COPAN S.r.L.**
**Via Trento**
**I-25060 Collebeato Brescia (IT)**

(72) Inventor: **Triva, Giorgio**
**Via Trento**
**I-25060 Collebeato (Brescia) (IT)**

**Triva, Daniele**
**Via Trento**
**I-25060 Collebeato (Brescia) (IT)**

(74) Representative: **Manzoni, Orazio**
**Ufficio Internazionale Brevetti MANZONI & MANZONI**
**Piazzale Arnaldo 2**
**I-25121 Brescia (IT)**

(54) Tampon with a reservoir of transfer medium to take specimens of substances to analyse.

㊼ A tampon to take specimens of substances to analyse and place them into a test tube. The tampon is supported by a small rod (4) fixed into a grip or handle (5) acting both as a stopper for the test tube (2) and as a support for a compressible, perforable or destructible reservoir (6) containing a liquid transfer medium (7) delivered along said rod as soon as the reservoir is compressed or broken.

Fig.2

EP 0 223 745 A1

## Description

### TAMPON WITH A RESERVOIR OF TRANSFER MEDIUM TO TAKE SPECIMENS OF SUBSTANCES TO ANALYSE

The present invention relates to a tampon with a reservoir of transfer medium to take specimens of substance to analyse and place them into a test tube.

### STATE OF PRIOR ART

Tampons normally used to take specimens of organic substances to analyse, like bacteria, germs, etc. from body cavities, are generally composed of a swab of cottonwool applied to the end of a rod made of metal, wood or plastic material, its opposite end being fitted with a grip or handle. After taking the specimen the tampon is placed into a container or test tube with a transfer medium which will feed the substance collected in the tampon.

As specified for a known execution the transfer medium is usually a gelatinous matter directly placed on the bottom of the test tube which is then closed and sealed to ensure its sterility. The sealed test tube is then packed in a protective wrapping together with the rod fitted with its tampon and grip. After taking the specimen the test tube is opened and the tampon inserted, its grip closing the test tube like a stopper.

In another known execution, on the bottom of the test tube there are two compartments, one for the still gelatinous transfer medium and the other one for the tampon before it is used. In this case tampon and test tube can be placed one into the other and packed together, the grip of the tampon primarily closing the test tube and also sealing said tube when the specimen has been taken and placed into the transfer medium.

Still another known execution comprises a vial containing the preferably liquid transfer medium which is placed on the bottom of the test tube; the tampon is fitted into the test tube and its grip is used to close the test tube both to seal the new tube and to close it after taking the specimen and placing it into the test tube. In this case the vial is broken to let out the transfer medium into the test tube when the tampon is replaced into the test tube. It is however necessary to prevent the vial from breaking by accident or too soon and to this purpose it has to be protected by a suitable collar or the like, but this of course increases production costs.

Altogether in the known executions the transfer medium for the specimens taken is always placed either directly or indirectly on the bottom of the test tube, thus causing some problems of containment and protection and the tampon is brought into contact with the transfer medium after the specimen has been taken. In addition, the grip of the tampon besides serving to handle the tampon is just used to close and seal the test tube at least during preservation and transport of specimens.

Prior art has also proposed an execution where the transfer medium is not directly placed into the test tube but into a separate container or vial. Originally, the container or vial is fitted to the handle or grip of the tampon which is introduced and stored in a protective cap fastened in turn to the grip on the end which is opposite to the vial.

It is however quite a complicated contrivance, which is also expensive and not too easy to use as several components are to be handled.

In this item the grip of the tampon is acting as a double stopper to close the vial at one end and the protective cap at the other end with a double sealing to ensure sterility. To use the tampon it has to be removed from the protective cap and after taking the specimen it is introduced into the vial after removing it from the grip and taking care to slide the grip along the rod of the tampon till the vial is closed.

### SPECIFICATION OF THE INVENTION

The present invention is instead related to a tampon to take and transfer specimens of substances to be analysed comprising a rod with a grip incorporating a reservoir filled with a transfer medium to feed the substance taken in through the rod of the tampon.

It is therefore an object of the invention to propose a tampon to take specimens of substances to be analysed in which the transfer medium is delivered to its swab starting from its grip und using its rod as a conduit to take the medium to the substance to be fed.

Another object of the invention is to propose a tampon to be used as specified above consenting an easy delivery of the transfer medium without letting go the grip and without being obliged to tamper with the tampon or the test tube.

Still another object of the invention is to propose a unit consisting of tampon and test tube to take and store substances to analyse of simple design, low production cost, easy to use and where the test tube does not contain the transfer medium, its only purpose being to store the tampon before and after its use while the grip of the tampon is closing it in both conditions.

Still another object of the invention is to propose a tampon fitted with a compressible reservoir for the transfer medium compelling said medium to flow along the tampon rod while it is delivered.

The present invention, thus relates to a tampon according to Claim 1.

More details of the invention will be set forth in the following specification and will become apparent from the enclosed drawing where:

Figure 1 is a view of the tampon and the test tube separated from each other;

Figure 2 shows a longitudinal section of the assembled device, the tampon grip housing a radially compressible reservoir;

Figure 3 shows a section like that in Figure 2, but with a tampon grip comprising an axially compressible reservoir with perforable bottom; and

Figures 4 and 5 show cross sections of two different types of tampon rod.

Referring to the enclosed drawing reference numbers 1 and 2 indicate a tampon to take specimens and a test tube to store said tampon before and after its use, with some cottonwool 2' on the bottom of the test tube if necessary.

Tampon 1 comprises a rod 4 with a swab 3 applied to one end while its opposite end is fitted with a grip 5. Said grip is also designed to plug test tube 2 and to bear a reservoir 6 containing, either directly or indirectly a liquid transfer medium 7.

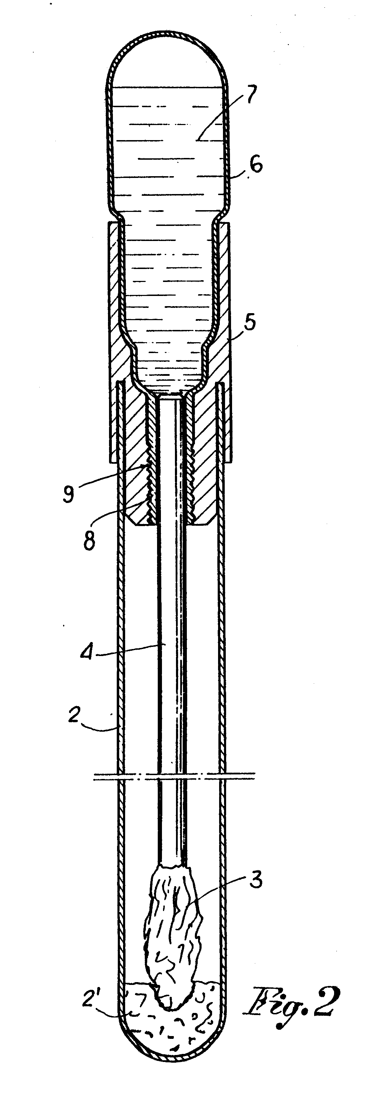

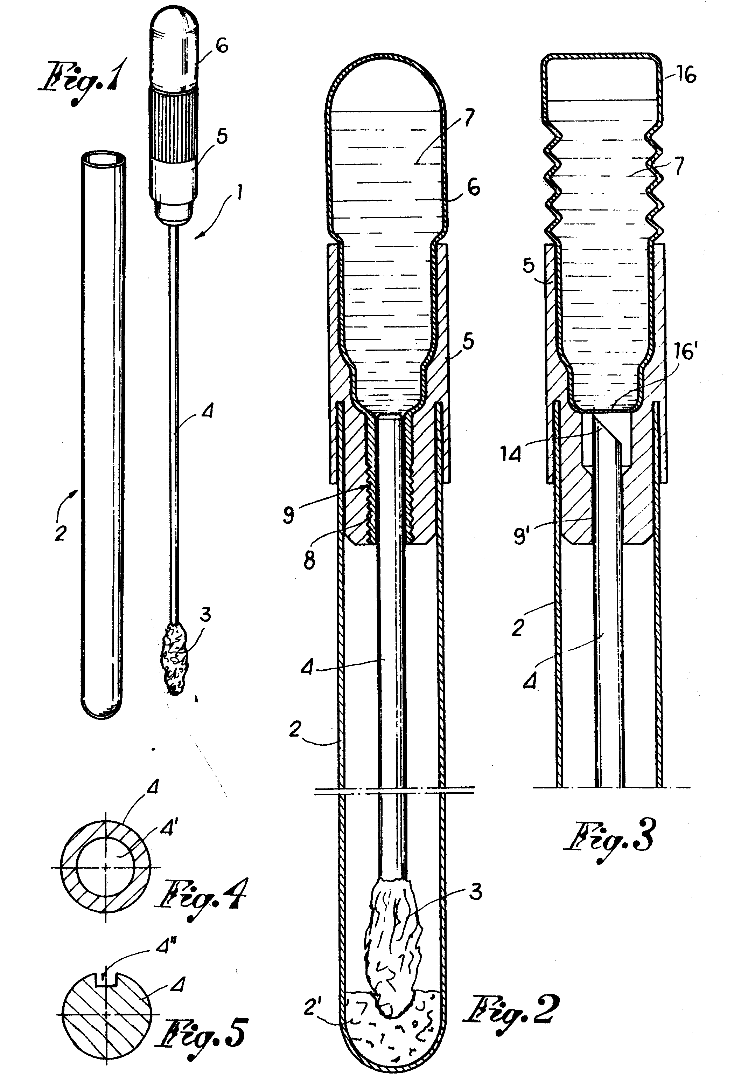

Figure 2 shows an execution of the invention with reservoir 6 made of a plastic and elastic material projecting at least partially from grip 5 and compressible like a rubber syringe. Reservoir 6 contains the transfer medium 7 and its tapered neck 8 is fitted, either by screwing or by pressure, into an axial hole 9 in grip 5. The top end of rod 4 opposite to the one with swab 3 is lodged in neck 8 of reservoir 6 so as to contact transfer medium 7.

Rod 4 of tampon 1 may be made of metal, wood or plastics and have an axial through hole 4' as shown in Figure 4 giving it a tubular shape, but it may also have a longitudinal recess or groove 4" as shown in Figure 5 of the drawing. If it is tubular, in rod 4 one or more radial holes may be bored.

To begin with tampon 1 is stored and protected in test tube 2 which is closed and sealed by grip 5 acting as a stopper and transfer medium 7 is stored in reservoir 6 assembled to said grip. The moment it has to be used tampon 1 is removed from test tube 2 and after taking the specimen it is replaced into the tube.

When compressing reservoir 6 transfer medium 7 will flow along rod 4 and finally reach swab 3 to feed the collected substance. Thus rod 4 is acting as a conduit to take the medium to the substance in the swab, the medium flowing down either through hole 4' or through groove 4".

In the variant shown in Figure 3 of the drawing grip 5 of tampon 1 is holding a reservoir 16 of transfer medium which is compressible like bellows, its bottom 16' being perforable. Rod 4 is fixed into an axial hole 9' in grip 5 and its end is chamfered to a tip 14 in front of the perforable bottom 16' of reservoir 16.

After taking the specimen with the tampon the operator has only to compress reservoir 16 in order to get bottom 16' perforated and the transfer medium will flow down along the rod and to the substance to be fed.

Still remaining within the limits of the invention the reservoir may be differently shaped and contain the transfer medium either directly as specified above or indirectly, i.e. in a vial to be broken by compressing the reservoir or the grip in order to deliver the medium and let it flow down to the substance to be fed in the swab 3.

The outlet of the transfer medium from the reservoir may also be displaced and not connected to rod 4. In this case rod 4 will simply act as a holder of the swab or tampon while the transfer medium will drop on the swab without flowing through the rod or come down along a groove eventually cut into the wall of the test tube.

## Claims

1.) A tampon to take and transfer into a test tube specimens of substances to be analysed, comprising a swab of cottonwool (3) fitted to one end of a rod (4) the opposite end of said rod being fitted with a grip (5) also acting as a stopper to close said test tube, said tampon being characterized in that the grip (5) incorporates a reservoir (6, 16) containing a liquid transfer medium (7, 17) which is delivered along said rod (4) to said swab (3) when said reservoir is either compressed or broken.

2.) Tampon according to claim 1) wherein said reservoir (6) consists of an elastic body fitted to grip (5) of rod (4) and has a radially compressible part projecting above the grip.

3.) Tampon according to claim 1) wherein said reservoir (16) consists of an elastic body fitted to grip (5) of rod (4) and has an axially compressible part projecting above the grip.

4.) Tampon according to claim 2) or 3) wherein said reservoir has a tapered neck (8) receiving the top end of rod (4) to bring it into contact with the transfer medium stored in said reservoir.

5.) Tampon according to claims 2) or 3) wherein said reservoir (16) has a perforable bottom (16') and wherein the rod (4) has a chamfered or at any rate sharp-edged tip (14) pointed at the perforated bottom of the reservoir so as to open it when its compressible portion is pressed together.

6.) Tampon according to any one of the previous claims in which the transfer medium is directly contained in said reservoir (6, 16) while its rod (4) is used as a conduit to take the transfer medium to the substance to be fed owing to a compression of the reservoir.

7.) Tampon according to any one of the claims 1) to 6) wherein the transfer medium is contained in a breakable vial inside said reservoir, while its rod (4) is used as a conduit to take the transfer medium to the substance to be fed after the vial has been broken owing to a compressione of the reservoir.

8.) Tampon according to any one of the preceding claims wherein rod (4) is comprising either a lateral groove or a longitudinal hole (4", 4').

9.) Tampon according to claim 1) in which the reservoir is a cavity delimited by a com pressible portion of grip (5) on rod (4), said rod (4) extending into said grip and projecting into the cavity used as a reservoir.

10.) Tampon to take and transfer into a test tube specimens of substances to be analysed, comprising a cottonwool swab applied to one end of a rod (4) fitted with a grip (5) at its opposite end, said grip also acting as a stopper to close said test tube, characterized in that said grip comprises a reservoir containing a transfer medium delivered into the test tube when said reservoir is either compressed or broken, said transfer medium readily flowing into the test tube or along at least one groove recessed into the wall of the test tube.

0223745

*Fig.2*

0223745

Fig.1

Fig.4

Fig.5

Fig.3

Fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 918 435 (BEALL et al.)<br><br>* Figures 1-5; column 2, lines 1-45; column 3, line 25 - column 4, line 22 * | 1-4,7-10 | A 61 F 13/20<br>A 61 B 10/00 |
| Y | | 5,6 | |
| Y | US-A-4 407 283 (REYNOLDS)<br>* Figures 2,4A * | 5,6 | |
| X | GB-A-1 453 961 (MEDEX INC.)<br><br>* Page 1, lines 68-73; figure 1 * | 1,2,6-8,10 | |
| A | GB-A- 477 750 (A.E. MASSMAN)<br>* Figure 3 * | 1 | TECHNICAL FIELDS SEARCHED (Int. Cl.4)<br><br>A 61 F<br>A 61 M |

The present search report has been drawn up for all claims

| Place of search | Date of completion of the search | Examiner |
|---|---|---|
| THE HAGUE | 04-02-1987 | ARGENTINI A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82